# EUROPÄISCHE PATENTSCHRIFT

(11) **EP 1 314 443 B1**
(45) Veröffentlichungstag und Bekanntmachung des Hinweises auf die Patenterteilung: **17.01.2007**
(21) Anmeldenummer: 02025199.7
(22) Anmeldetag: 11.11.2002
(51) Int. Cl.: A61M 1/36, A61M 1/14

(54) **Vorrichtung zur Behandlung einer medizinischen Flüssigkeit**
Device for treating a medical fluid
Dispositif pour le traitement d'un fluide médical

(30) Priorität: 26.11.2001 DE 10157924
(43) Veröffentlichungstag der Anmeldung: 28.05.2003
(73) Patentinhaber: Fresenius Medical Care Deutschland GmbH, 61352 Bad Homburg v.d.H. (DE)
(72) Erfinder: Beden, Josef, 55252 Mainz-Kastel (DE); Herklotz, Martin, 61350 Heusenstamm (DE); Scherer, Jörg, 73432 Aalen (DE); Schneider, Hans-Peter, 61267 Neu Anspach (DE)
(74) Vertreter: Laufhütte, Dieter

(56) Entgegenhaltungen:
- WO-A-01/17606
- DE-A- 19 837 667
- US-A- 5 938 634

## Beschreibung

Die Erfindung betrifft eine Vorrichtung zur Behandlung einer medizinischen Flüssigkeit nach dem Oberbegriff des Anspruchs 1.

Derartige Vorrichtungen sind weithin bekannt und bestehen aus einer fest installierten Flüssigkeitsbehandlungsmaschine, einer Kassette mit flüssigkeitsführenden Kanälen, die mittels einer Folie verschlossen sind, und einer zwischen diesen beiden angeordneten elastischen Matte. Als medizinische Flüssigkeiten können beispielsweise Blut oder aber auch Dialysier-Flüssigkeiten für die Peritoneal- sowie für die Hämodialyse behandelt werden. Die entsprechenden Kassetten sind zweckmäßigerweise als Wegwerfteil ausgebildet. Eine derartige Flüssigkeitsbehandlungsmaschine kann beispielsweise auch eine Vorrichtung zur Messung von Parametern medizinischer Flüssigkeiten sein, wie sie in der DE 198 37 667 A1 beschrieben wurde.

In diesen Vorrichtungen wird zwischen der elastischen Matte und der Kassette ein Unterdruck angelegt, um eine Verformung der Folie bei Unterdruck im Kanal, die zur Kanalverengung in der Kassette führt, zu verhindern, um an Zugabestellen für die medizinische Flüssigkeit die Folie abzuheben und damit den Zugang freihalten zu können, um eine Luftcompliance in der Pumpeneinrichtung zu vermeiden und um an speziellen Sensorpositionen eine luftfreie Ankopplung zwischen Sensoroberfläche und Folie gewährleisten zu können.

Die Luftabsaugung bedingt maschinenseitige Öffnungen und eine daran angeschlossene Absaugeinheit, beispielsweise eine Vakuumpumpe, wobei die Vakuumverteilung möglichst gleichmäßig und sicher über die gesamte Folienfläche der Kassette gewährleistet sein soll.

Aufgabe der vorliegenden Erfindung ist es, die gattungsgemäße Vorrichtung derart weiterzubilden, daß im Betrieb eine störungsfreie Luftabsaugung ermöglicht wird, wobei hier das Vakuum über die Fläche der elastischen Matte hin gleichmäßig verteilt werden soll und eine einfache und störungsfreie Luftentfernung möglich sein soll, während im Ruhezustand die zur Luftentfernung notwendigen Öffnungen zumindest weitgehend verschlossen sein sollen.

Erfindungsgemäß wird diese Aufgabe durch die Merkmalskombination des Anspruchs 1 gelöst. Demnach sind in der elastischen Matte Mattenkanäle ausgenommen, die entlang flüssigkeitsführender Kanäle der Kassette verlaufen. Die Mattenkanäle erreichen von einem Vakuumanschluss der elastischen Matte zur Ansaugeinrichtung ausgehend alle wichtigen Stellen der elastischen Matte. Diese Mattenkanäle liegen, um Undichtigkeiten bei der Anpressung der Kanalwülste der aufliegenden Kassette zu vermeiden, innerhalb einer Kanalstruktur der Kassette. Nur dort, wo sich auch innerhalb der Kassette ein kassettenseitiger Kanal bzw. eine sonstige, flüssigkeitsführende Struktur befindet, ist auch in der entsprechenden elastischen Matte ein Mattenkanal vorgesehen. In den Mattenkanälen sind Schlitze ausgeführt. Diese in der elastischen Matte vorgesehenen Schlitze verhalten sich ähnlich wie eine Lippendichtung. Beim Anlegen des Vakuums kann Luft zwischen der Flüssigkeitsbehandlungsmaschine und der Kassette abgesaugt werden, da die Randbereiche der Schlitze aufgrund des Vakuums und der Freigabe einer Öffnung eingezogen werden. Aufgrund der Rückstellkraft des elastischen Materials schließen sich aber die Schlitze sofort, wenn das Anlegen des Vakuums unterbrochen wird, d.h. wenn sich die Vorrichtung im Ruhezustand befindet oder wenn kein nennenswerter Differenzdruck herrscht. In diesem Zustand wird ein Flüssigkeitseintritt sicher verhindert. Dagegen kann bei geöffneten Schlitzen der Zugang zur Absaugeinrichtung, d.h. die Weiterführung von der Matte zur Vakuumpumpe gegen fehlerhaften Flüssigkeitszutritt mittels geeigneter Maßnahmen (z.B. Filter) geschützt werden.

Bevorzugte Ausgestaltungen der Erfindung ergeben sich aus den sich an den Hauptanspruch anschließenden Unteransprüchen.

So können die Schlitze entlang der Mattenkanäle immer wieder für kurze Bereiche unterbrochen sein. Hierdurch wird den Schlitzen eine ausreichende Stabilität verliehen, die die Ventilfunktion unterstützen.

An bestimmten Stellen ist eine Vakuumweiterleitung mittels der innerhalb der Kassettenkanäle verlaufenden Mattenkanäle nicht möglich, da beispielsweise Ausnehmungen in der elastischen Matte vorgesehen sind, die die Mattenkanäle unterbrechen. In diese Ausnehmungen greifen beispielsweise maschinenseitige Pumpenmembranen oder Ventile ein. Diese Strukturierung würde dazu führen, daß eine störungsfreie Luftabsaugung durch die Mattenkanäle nicht mehr erfolgen kann. Um dennoch einen möglichst dichten flächenartigen Kontakt zwischen der Kassette und der Maschine zu schaffen und um die hierfür notwendige Unterdruckquelle effizient an die verschiedenen Bereiche der Kontaktfläche zu leiten, sind hier zusätzliche im Vergleich zu den Mattenkanälen flachere Verbindungskanäle ausgebildet, die diese größerflächigen Ausnehmungen innerhalb der elastischen Matte umgehen. Diese Verbindungskanäle sind so flach und schmal ausgebildet, daß das umliegende Mattenmaterial der elastischen Matte genügend Eigenstabilität aufweist, um ein Verformen und Verschließen des Verbindungskanals auch bei Anlegen des Vakuums und Verpressung zu verhindern. Bedingt durch diese bevorzugte Ausgestaltung ist sichergestellt, daß die Schlitze und Kanäle bei Verpressen und Anlegen von Vakuum offen bleiben und die Matte ausreichend stabil bleibt, wobei die Dichtungswirkung der Matte aufrechterhalten bleibt und die zur Verfügung gestellte Kanalstruktur für den abzusaugenden Luftvolumenstrom ausreicht.

Schließlich ist die elastische Matte in bevorzugter Art und Weise auswechselbar ausgestaltet.

Weitere Einzelheiten und Vorteile der Erfindung werden anhand eines in der Zeichnung dargestellten Ausführungsbeispiels näher erläutert. Es zeigen:
- Fig. 1:: eine schematische dreidimensionale Darstellung eines Ausschnitts einer elastischen Matte nach einer Ausführungsform der vorliegenden Erfindung,
- Fig. 2:: einen Schnitt entlang der Schnittlinie A-A',
- Fig. 3:: einen Schnitt durch die Schnittlinie B-B' und
- Fig. 4:: einen Schnitt entsprechend der Schnittlinie C-C'.

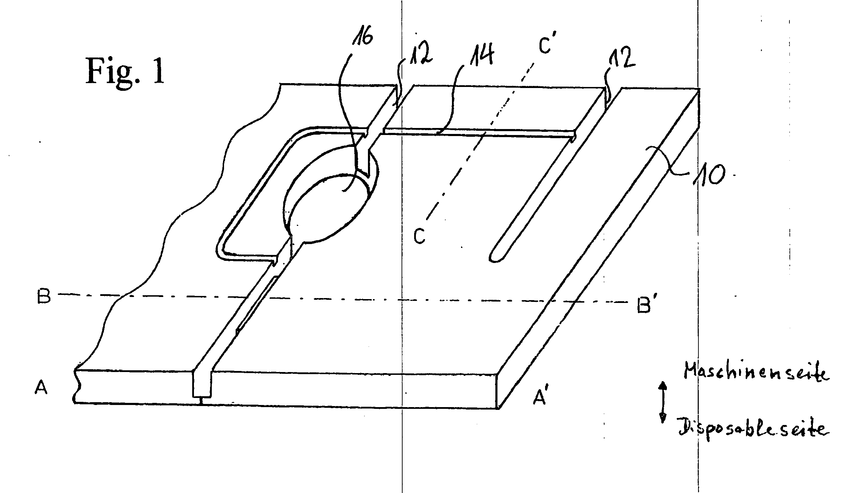

In Fig. 1 ist eine elastische Matte 10 schematisch dargestellt, die zwischen einer hier nicht näher dargestellten Flüssigkeitsbehandlungsmaschine und einer meist als Wegwerfteil ausgebildeten Kassette (Disposable) angeordnet ist. Auf der sogenannten Maschinenseite, also auf der Oberfläche, die im eingebauten Zustand zur Flüssigkeitsbehandlungsmaschine weist, sind Mattenkanäle 12 und Verbindungskanäle 14 ausgebildet. Weiterhin ist in der elastischen Matte 10 eine Ausnehmung 16 angeordnet, in welche beispielsweise im montierten Zustand ein maschinenseitiges Ventil rundumdichtend eingreift. Es ist leicht nachvollziehbar, daß dieses maschinenseitige Ventil den entsprechenden Mattenkanal 12, der jeweils in die Öffnung 16 einmündet, unterbricht. Um hier dennoch eine Luftabsaugung zu ermöglichen, ist ein Verbindungskanal 14 vorgesehen, der die beiden unterbrochenen Äste des Mattenkanals 12 miteinander und diese wiederum mit einem weiteren, parallelen Mattenkanal 12 verbindet. Diese hier dargestellte Struktur ist natürlich nur exemplarisch und kann in beliebiger Weise verändert werden. Während auf der Maschinenseite der elastischen Matte 10 die Kanalstrukturen vorgesehen sind, ist die Disposableseite, also die Seite, die zur Kassette gerichtet ist, glatt, d.h. eben ausgeführt.

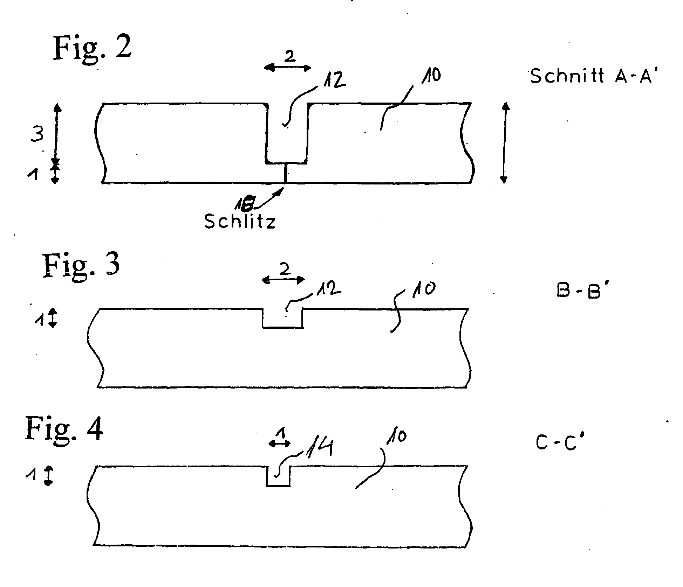

Anhand der Schnittdarstellungen der Figuren 2 bis 4 kann die Struktur der einzelnen Kanäle näher erläutert werden. In Fig. 2 ist der Schnitt A-A' gemäß Fig. 1 gezeigt, bei dem ein Mattenkanal sichtbar wird, der bei der hier verwendeten elastischen Matte mit einer Dicke von 4 mm eine Tiefe von 3 mm und eine Breite von 2 mm aufweist. In dem verbleibenden Mattenbereich unterhalb des Kanals 12, der eine Dicke von 1 mm aufweist, ist ein Schlitz 18 angeordnet, der eine Art Ventilfunktion übernimmt. Beim Anlegen von Vakuum öffnen sich die beiden im Schlitz 18 anstoßenden Bereiche der elastischen Matte 10 und ermöglichen die Absaugung von Luftgas. Im Ruhezustand, also nach Abschaltung des Vakuums bzw. bei Einstellung des Gleichgewichtszustandes kehren die beiden seitlichen Bereiche wieder in ihre Ausgangsstellung zurück und verschließen die Öffnung. Um diese Rückstellwirkung zu unterstützen sind im Kanal 12 Bereiche zwischen Schlitzen 18 vorgesehen, die zum einen nicht geschlitzt sind und zum anderen im Bereich des Kanals 12 weniger tief ausgeschnitten sind. Ein entsprechender Bereich ergibt sich aus dem Schnitt B-B' gemäß Fig. 3, aus welchem hervorgeht, daß der Kanal 12 in diesem Bereich zwar die gleiche Breite von 2 mm aufweist, demgegenüber aber nur 1 mm tief ist.

In der Darstellung gemäß Fig. 4 ist in der Schnittdarstellung von C-C' ein Verbindungskanal 14 gezeigt, der, wie hier deutlich zu sehen ist, schmaler und weniger tief als der Mattenkanal 12 ausgenommen ist. Hier ist jeweils die Breite des Verbindungskanals 14 ein Millimeter und die Tiefe jeweils auch ein Millimeter.

Mit der elastischen Matte gemäß dieser Erfindung ist es sichergestellt, daß der Flüssigkeitsbehandlungsmaschineninnenraum im Ruhezustand durch den Selbstverschluß der Schlitze 18 geschützt ist. Gleichzeitig wird eine gleichmäßige und flächendeckende Luftabsaugung zwischen der Flüssigkeitsbehandlungsmaschine und der Kassette erreicht, da hier eine Vielzahl von Schlitzen 18 parallel abgesaugt wird. Somit kann ein geringfügiger Verschluß auch ohne nachteilige Folgen für andere Bereiche sein.

Bei einer dünnen Matte 10, wie sie beispielsweise im Ausführungsbeispiel vorgestellt wurde, kann beim Anlegen von Vakuum der Öffnungseffekt der Schlitze genutzt werden.

Da die elastische Matte 10 austauschbar ist, kann diese nach Verunreinigung oder Defekt leicht erneuert werden. Es ist besonders vorteilhaft, daß keine Strukturen in den maschinenseitigen festen Teilen notwendig sind. Auf der Maschinenseite der elastischen Matte 10 können offene Strukturen ausgebildet sein, so daß keine Untertunnelungen oder geschlossene Strukturen notwendig sind. Demgegenüber ist die Seite der elastischen Matte 10, die zur Kassette gerichtet ist, als weitgehend glatte, geschlossene Oberfläche ausgebildet, die sich z.B. leicht reinigen läßt.

## Patentansprüche

1. Vorrichtung zur Behandlung einer medizinischen Flüssigkeit, bestehend aus einer fest installierten Flüssigkeitsbehandlungsmaschine, einer Kassette mit flüssigkeitsführenden Kanälen und einer zwischen der Flüssigkeitsbehandlungsmaschine und den flüssigkeitsführenden Kanälen angeordneten elastischen Matte (10),
**dadurch gekennzeichnet,**
**daß** in der elastischen Matte (10) Mattenkanäle (12) ausgenommen sind, die entlang flüssigkeitsführender Kanäle der Kassette verlaufen und daß in den Mattenkanälen (12) Schlitze (18) ausgeführt sind, die von den Mattenkanälen (12) zu der der Kassette zugewandten Seite verlaufen.

2. Vorrichtung nach Anspruch 1, **dadurch gekennzeichnet, daß** die Schlitze (18) entlang der Mattenkanäle (12) immer wieder für kurze Bereiche unterbrochen sind.

3. Vorrichtung nach Anspruch 2, **dadurch gekennzeichnet, daß** die Mattenkanäle (12) in den geschlitzten Bereichen tiefer ausgenommen sind als in den nicht geschlitzten Bereichen.

4. Vorrichtung nach einem der Ansprüche 1 bis 3, **dadurch gekennzeichnet, daß** die elastische Matte (10) im Vergleich zu den Mattenkanälen (12) flachere Verbindungskanäle (14) aufweist, insbesondere zur Umgehung von Durchbrüchen in den Mattenkanälen (12), die beispielsweise der Aufnahme von maschinenseitigen Pumpenmembranen und Ventilen dienen.

5. Vorrichtung nach einem der Ansprüche 1 bis 4, **dadurch gekennzeichnet, daß** die elastische Matte (10) auswechselbar ist.

6. Vorrichtung nach einem der Ansprüche 1 bis 5, **dadurch gekennzeichnet, daß** die elastische Matte (10) im Randbereich der Kassette durch Verpressen eines im Bereich der Kassette umlaufenden Dichtrandes abdichtbar ist.

7. Vorrichtung zur Behandlung einer medizinischen Flüssigkeit, bestehend aus einer fest installierten Flüssigkeitsbehandlungsmaschine und einer elastischen Matte (10) zur Ankopplung einer Kassette mit flüssigkeitsführenden Kanälen,
**dadurch gekennzeichnet,**
**dass** in der elastischen Matte (10) Mattenkanäle (12) ausgenommen sind, die entlang flüssigkeitsführender Kanäle der ankoppelbaren Kassette verlaufen und daß in den Mattenkanälen (12) Schlitze (18) ausgeführt sind, die von den Mattenkanälen (12) zu der der ankoppelbaren Kassette zugewandten Seite verlaufen.

8. Elastische Matte zur Verwendung in einer Vorrichtung nach einem der Ansprüche 1 bis 6.,
**dadurch gekennzeichnet,**
**daß** in der elastischen Matte (10) Mattenkanäle (12) ausgenommen sind, die entlang flüssigkeitsführender Kanäle der Kassette verlaufen und daß in den Mattenkanälen (12) Schlitze (18) ausgeführt sind, die von den Mattenkanälen (12) zu der der Kassette zugewandten Seite verlaufen.

## Claims

1. A device for a treatment of a medical fluid, comprising a fixedly installed fluid treatment machine, a cassette with fluid-carrying passages and an elastic mat (10) arranged between the fluid treatment machine and the fluid-carrying passages,
**characterised in that**
mat passages (12) are recessed in the elastic mat (10), said mat passages extending along fluid-carrying passages of the cassette, and that provided in the mat passages (12) are slits (18) which extend from the mat passages (12) to the side which is towards the cassette.

2. A device according to claim 1 **characterised in that** the slits (18) along the mat passages (12) are repeatedly interrupted for short regions.

3. A device according to claim 2 **characterised in that** the mat passages (12) are recessed more deeply in the slitted regions than in the on-slitted regions.

4. A device according to one of claims 1 to 3 **characterised in that** the elastic mat (10) has shallower connecting passages (14) in comparison with the mat passages (12), in particular for circumventing interruptions in the mat passages (12) which for example serve to receive machine-side pump diaphragms and valves.

5. A device according to one of claims 1 to 4 **characterised in that** the elastic mat (10) is interchangeable.

6. A device according to one of claims 1 to 5 **characterised in that** the elastic mat (10) can be sealed off in the edge region of the cassette by pressing against a peripherally extending sealing edge in the region of the cassette.

7. A device for the treatment of a medical fluid comprising a fixedly installed fluid treatment machine and an elastic mat (10) for coupling a cassette with fluid-carrying passages,
**characterised in that**
mat passages (12) are recessed in the elastic mat (10), the mat passages extending along fluid-carrying passages of the coupleable cassette, and that provided in the mat passages (12) are slits (18) which extend from the mat passages (12) to the side which is towards the coupleable cassette.

8. An elastic mat for use in a device according to one of claims 1 to 6,
**characterised in that**
mat passages (12) are recessed in the elastic mat (10), said mat passages extending along fluid-carrying passages of the cassette, and that provided in the mat passages (12) are slits (18) which extend from the mat passages (12) to the side which is towards the cassette.

## Revendications

1. Dispositif pour le traitement d'un liquide médical, comprenant une machine de traitement de liquide installée de façon fixe, une cassette avec des canaux véhiculant du liquide et un tapis (10) élastique disposé entre la machine de traitement de liquide et les canaux véhiculant du liquide,
**caractérisé en ce que**
dans le tapis (10) élastique sont évidés des canaux pour tapis (12), qui sont agencés le long de canaux véhiculant du liquide de la cassette et **en ce que** dans les canaux pour tapis (12) sont réalisées des fentes (18), qui sont agencées depuis les canaux pour tapis (12) vers le côté opposé à la cassette.

2. Dispositif selon la revendication 1, **caractérisé en ce que** les fentes (18) sont interrompues le long des canaux pour tapis (12) sans cesse pour de courtes zones.

3. Dispositif selon la revendication 2, **caractérisé en ce que** les canaux pour tapis (12) sont évidés plus profondément dans les zones fendues que dans les zones non fendues.

4. Dispositif selon l'une quelconque des revendications 1 à 3, **caractérisé en ce que** le tapis (10) élastique présente par rapport aux canaux pour tapis (12) des canaux de liaison (14) plus plats, en particulier pour contourner des percements dans les canaux pour tapis (12), qui servent par exemple au logement de diaphragmes de pompe côté machine et de vannes.

5. Dispositif selon l'une quelconque des revendications 1 à 4, **caractérisé en ce que** le tapis (10) élastique est interchangeable.

6. Dispositif selon l'une quelconque des revendications 1 à 5, **caractérisé en ce que** le tapis (10) élastique peut être rendu étanche dans la zone périphérique de la cassette par compression d'un bord d'étanchéité périphérique dans la zone de la cassette.

7. Dispositif pour le traitement d'un liquide médical, comprenant une machine de traitement du liquide installée de façon fixe et un tapis (10) élastique pour le rattachement d'une cassette avec des canaux véhiculant du liquide,
**caractérisé en ce que**
dans le tapis (10) élastique sont évidés des canaux pour tapis (12), qui sont agencés le long de canaux véhiculant du liquide de la cassette pouvant être couplée et **en ce que** dans les canaux pour tapis (12) sont réalisées des fentes (18), qui sont agencées depuis les canaux pour tapis (12) vers le côté tourné vers la cassette pouvant être accouplée.

8. Tapis élastique pour l'utilisation dans un dispositif selon l'une quelconque des revendications 1 à 6,
**caractérisé en ce que**
dans le tapis (10) élastique sont évidés des canaux pour tapis (12) qui sont agencés le long des canaux véhiculant du liquide et **en ce que** dans les canaux pour tapis (12) sont réalisées des fentes (18) qui sont agencées depuis les canaux pour tapis (12) vers le côté tourné vers la cassette.
